# FASCICULE DE BREVET EUROPEEN

(11) **EP 2 793 683 B1**
(45) Date de publication et mention de la délivrance du brevet: **25.11.2015**
(21) Numéro de dépôt: 12810360.3
(22) Date de dépôt: 06.12.2012
(51) Int. Cl.: A61B 3/15, A61B 3/028, A61B 3/036, A61B 3/103, G02C 7/06, A61B 3/00

(54) **DISPOSITIF DE DÉTERMINATION D'AU MOINS UN PARAMÈTRE DE VISION D'UN SUJET SUIVANT UNE PLURALITÉ DE DIRECTIONS DE VISÉE**
VORRICHTUNG ZUR BESTIMMUNG VON WENIGSTENS EINEM SICHTPARAMETER EINER PERSON IN MEHREREN BLICKRICHTUNGN
DEVICE FOR DETERMINING AT LEAST ONE SIGHT PARAMETER OF A SUBJECT IN A PLURALITY OF VIEWING DIRECTIONS

(30) Priorité: 22.12.2011 FR 1104036
(43) Date de publication de la demande: 29.10.2014
(73) Titulaire: Essilor International (Compagnie Générale d'Optique), 94220 Charenton-le-Pont (FR)
(72) Inventeur: BARANTON, Konogan, F-94220 Charenton-le-Pont (FR); ESCALIER, Guilhem, F-94220 Charenton-le-Pont (FR)
(74) Mandataire: Chauvin, Vincent
(86) Numéro de dépôt international: PCT/FR2012/052821
(87) Numéro de publication internationale: WO 2013/093279

(56) Documents cités:
- EP-A1- 1 882 444
- EP-A2- 0 326 760
- US-A- 3 982 827
- US-A- 5 444 504
- US-A1- 2008 151 185

## Description

### DOMAINE TECHNIQUE AUQUEL SE RAPPORTE L'INVENTION

La présente invention concerne de manière générale le domaine des dispositifs d'optométrie. Plus particulièrement, l'invention concerne un appareil d'optométrie pour déterminer les différentes valeurs de la prescription d'une lentille de lunettes de compensation visuelle multifocale ou progressive, ou de lunettes destinées à la compensation en vision de près (readers), liées à la mesure de la réfraction oculaire différenciée d'une pluralité de regards et en particulier en vision de loin et en vision de près. Ces mesures sont destinées à être exploitées pour la conception optique et la fabrication des faces de réfraction d'une lentille compensatrice de lunettes multifocale ou progressive, ou destinées à la compensation en vision de près (y compris lunettes de type readers, lunettes pré-montées), que ce soit des verres passifs ou de puissances optiques variables par commande électronique.

### ARRIERE-PLAN TECHNOLOGIQUE

Depuis une cinquantaine d'années, les lentilles compensatrices de lunettes multifocales et progressives ont connu un essor considérable. Une lentille compensatrice multi-focale présente au moins deux puissances compensatrices distinctes pour deux zones de la lentille compensatrice correspondant à deux distances de vision. Une lentille compensatrice progressive présente une puissance variable sur la surface de la lentille, allant, par exemple dans le cas d'une compensation de presbytie, d'une zone où la compensation sphérique est faible pour la vision de loin (VL) à une zone où la compensation sphérique est plus forte pour la vision de près (VP). Une lentille compensatrice progressive présente généralement une compensation moyenne pour une distance de vision intermédiaire entre la vision de loin et la vision de près. Les lentilles compensatrices de lunettes multifocales ou progressives permettent au sujet de bénéficier d'une compensation de puissance optique adaptée pour différentes distances de vision sans changer de lunettes. Afin de déterminer les paramètres d'une lentille compensatrice multi-focale ou d'une lentille compensatrice progressive, il existe des appareils d'optométrie monoculaires ou binoculaires pour mesurer la compensation optique à apporter en vision de près et en vision de loin. Un appareil d'optométrie basé sur la mesure de la réflexion et/ou réfraction d'un faisceau lumineux par un oeil permet ainsi de mesurer la compensation de puissance (ou sphère) différenciée VL/VP c'est-à-dire la compensation à apporter à l'oeil mesuré en vision de près et en vision de loin. Une lentille compensatrice multi-focale ou progressive peut corriger non seulement un défaut de puissance optique mais aussi d'autres défauts de vision et en particulier l'astigmatisme. Basés sur le même principe de mesure de réflexion et/ou réfraction oculaire, certains appareils d'optométrie permettent de mesurer les paramètres de compensation d'astigmatisme (cylindre et axe) et/ou les paramètres de compensation d'ordre supérieur (cf. norme ISO 24157 :2008 qui spécifie les méthodes normalisées permettant de consigner les aberrations de l'oeil humain).

Actuellement les mesures de réflexion et/ou réfraction oculaire différenciées VL/VP se font uniquement de façon manuelle. Un optométriste utilise une lunette d'essai pour déterminer les différentes valeurs de la prescription des lentilles compensatrices.

Dans les appareils classiques d'optométrie, un système optique intercalé sur l'axe oculaire adapte la puissance optique pour modifier la distance d'accommodation visuelle sur une cible, la ligne de visée du regard restant horizontale.

Dans les études actuelles sur la mesure de réfraction différenciée (VL/VP), nous sommes confrontés au problème de mesurer la réfraction en vision de près tout en suivant l'abaissement physiologique du regard qui accompagne cette vision. Le document de brevet EP1882444 décrit une méthode et un dispositif pour mesurer les caractéristiques visuelles d'un oeil selon différentes directions du regard, dans lequel un aberromètre est placé sur un support mobile en rotation de manière à incliner l'axe de mesure pour l'aligner suivant une direction du regard abaissé. Cependant, si l'on souhaite utiliser un appareil commercial actuel dans une direction naturelle d'abaissement ou d'élévation du regard, une difficulté technique apparaît (impossibilité dans certains cas) pour positionner la voie de mesure dans l'axe de regard naturel du sujet. On se trouve en effet confronté à des collisions entre la tête et l'appareil de mesure. D'autre part les éléments mécaniques des systèmes existants, en particulier les platines en translation pour le centrage, sont conçus pour fonctionner sur un plan horizontal.

Il n'existe pas actuellement d'appareil d'optométrie, de type auto-réfracteur ou aberromètre, permettant d'étudier le phénomène de réfraction différenciée en vision de loin et en vision de près suivant différentes directions d'un axe oculaire ou suivant différentes directions du regard.

### OBJET DE L'INVENTION

On cherche à améliorer la précision des mesures ophtalmologiques différenciées en fonction de la distance de vision du sujet et de la direction de visée monoculaire ou binoculaire du sujet, pour améliorer la compensation différenciée apportée selon les conditions de vision d'un sujet de lentilles compensatrices de lunettes multifocales ou progressives. En particulier, on cherche à effectuer des mesures d'astigmatisme différenciées en VL/VP. A titre complémentaire, on cherche à effectuer des mesures d'aberrations d'ordre supérieur également différenciées selon que le sujet soit en VL/VP.

Un des buts de l'invention est de fournir un dispositif d'optométrie pour effectuer une mesure (objective ou subjective) d'au moins un paramètre de vision différencié VLNP d'un sujet en fonction de la direction de visée monoculaire ou binoculaire.

L'invention vise à proposer un dispositif d'optométrie pour mesurer au moins un paramètre de vision suivant différentes directions de visée monoculaire ou binoculaire d'un sujet.

Afin de remédier à l'inconvénient précité de l'état de la technique, la présente invention propose un dispositif de détermination d'au moins un paramètre de vision d'un sujet suivant une pluralité de directions de visée monoculaire ou binoculaire du sujet, ledit dispositif comprenant un appareil de mesure ophtalmologique monoculaire ou binoculaire, comprenant des moyens de mesure ophtalmologique du paramètre de vision à déterminer aptes à émettre un faisceau optique d'éclairage et à recevoir un faisceau optique de mesure suivant au moins un axe optique de mesure aligné avec une direction prédéterminée de visée de l'oeil concerné, des moyens de stimulation visuelle aptes à générer un faisceau optique de stimulation suivant un axe optique de stimulation aligné avec ladite direction prédéterminée de visée de l'oeil concerné, et des moyens de support de tête aptes à recevoir la tête d'un sujet et à la maintenir dans une posture déterminée.

Étant définie une ellipse ayant un premier foyer sur ledit axe optique de mesure et un deuxième foyer, on propose un dispositif comprenant au moins un système d'alignement optique disposé entre les moyens de mesure ophtalmologique et l'oeil concerné, ledit système d'alignement optique étant apte à réfléchir lesdits faisceaux optiques d'éclairage et de mesure entre les foyers de l'ellipse. Selon l'invention, le dispositif comporte en outre des moyens de réglage aptes à modifier la position relative dudit système d'alignement optique par rapport aux moyens de support de tête de manière à amener le deuxième foyer au voisinage du centre de rotation de l'oeil concerné du sujet. Selon l'invention, ledit système d'alignement optique comprend des premiers moyens optiques réfléchissants et des seconds moyens optiques réfléchissants, lesdits premiers moyens optiques réfléchissants étant tangents à ladite ellipse en un premier point sur une première direction de visée et en au moins un autre point sur au moins une autre direction de visée de l'oeil concerné, et lesdits seconds moyens optiques réfléchissants étant montés mobiles en rotation autour du premier foyer entre une première position dans laquelle le système d'alignement optique aligne ladite première direction de visée avec l'axe optique de mesure et au moins une autre position dans laquelle le système d'alignement optique aligne ladite au moins une autre direction de visée de l'oeil concerné avec l'axe optique de mesure.

D'autres caractéristiques non limitatives et avantageuses du dispositif de détermination d'au moins un paramètre de vision d'un sujet suivant une pluralité de directions de visée monoculaire ou binoculaire du sujet conforme à l'invention sont les suivantes :
- lesdits premiers moyens optiques réfléchissants comportent un miroir sphérique, un miroir plan, une pluralité de miroirs plans, une lame dichroïque ou une pluralité de lames dichroïques ;
- lesdits premiers moyens optiques réfléchissants comprennent un premier miroir et ledit système d'alignement optique comprend des moyens de déplacement du premier miroir aptes à déplacer le premier miroir suivant une trajectoire prédéterminée en fonction de la direction de visée monoculaire ou binoculaire du sujet ;
- lesdits moyens de déplacement dudit premier miroir comprennent un système articulé à biellettes et/ou une came et/ou un système mécanique de guidage ;
- ladite trajectoire prédéterminée étant une trajectoire elliptique et ledit premier miroir étant orienté de manière à être tangent à ladite trajectoire elliptique ;
- lesdits premiers moyens optiques réfléchissants comprennent un premier miroir ayant une première position prédéterminée tangente à ladite ellipse en un premier point et un deuxième miroir ayant une deuxième position prédéterminée tangente à ladite ellipse en un autre point ;

Alternativement, lesdits premiers moyens optiques réfléchissants comprennent un miroir ellipsoïdal, ou de façon générale une surface optique telle que le conjugué optique du point Y soit le point E ; et lesdits seconds moyens optiques réfléchissants comprennent un second miroir de puissance optique non nulle, de manière à ce que le système d'alignement optique constitué du premier et du second miroir soit sensiblement afocal, c'est-à-dire afocal au premier ordre au sens de la sphère moyenne dans l'approximation de Gauss.

Selon certains aspects particuliers :
- lesdits seconds moyens optiques réfléchissants comportent un miroir plan ;
- ledit système d'alignement optique comprend un moyen d'orientation des seconds moyens optiques réfléchissants apte à faire pivoter les seconds moyens optiques réfléchissants autour du premier foyer de l'ellipse en fonction de la direction de visée monoculaire ou binoculaire ;
- ledit système d'alignement comprend des moyens d'inclinaison dudit système d'alignement optique, lesdits moyens d'inclinaison étant aptes à orienter le plan de l'ellipse autour d'un axe passant par ses foyers ;
- ledit système d'alignement comprend au moins une première position prédéterminée associée à une première direction de visée et au moins une autre position prédéterminée associée à ladite au moins une autre direction de visée ;
- ladite première direction de visée est une direction horizontale droit devant le sujet et ladite au moins une autre direction de visée correspond à une direction de visée en vision de près inclinée par rapport à l'horizontale ;
- le dispositif comprend une première position prédéterminée pour ledit au moins un premier angle d'abaissement du regard et une deuxième position prédéterminée pour ledit au moins un autre angle d'abaissement du regard ;
- les moyens de mesure ophtalmologique sont aptes à mesurer et à enregistrer au moins un paramètre de vision de type sphère, cylindre, axe, aberrations d'ordre supérieur, de kératométrie et/ou de topographie cornéenne, et/ou de diamètre de pupille dans une première direction de visée et dans au moins une autre direction de visée, et/ou une différence entre un paramètre de vision mesuré dans ladite première direction de visée et mesuré dans ladite au moins une autre direction de visée.

Selon un mode de réalisation particulier, ledit appareil de mesure ophtalmologique est un appareil binoculaire ayant un premier axe de mesure associé à l'oeil droit du sujet et un deuxième axe de mesure associé à l'oeil gauche du sujet, et ledit dispositif comprend :
- un premier système d'alignement optique selon l'un des modes de réalisation de l'invention disposé entre ledit appareil de mesure ophtalmologique binoculaire et l'oeil droit du sujet, et
- un deuxième système d'alignement optique selon l'un des modes de réalisation de l'invention disposé entre ledit appareil de mesure ophtalmologique binoculaire et l'oeil gauche dudit sujet.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente schématiquement un dispositif selon un premier mode de réalisation de l'invention ;
- la figure 2 représente schématiquement un dispositif selon une première variante du premier mode de réalisation de l'invention;
- la figure 3 représente schématiquement un dispositif selon une deuxième variante du premier mode de réalisation de l'invention ;
- la figure 4 représente schématiquement un dispositif selon un deuxième mode de réalisation de l'invention ;
- la figure 5 représente schématiquement un dispositif selon un troisième mode de réalisation de l'invention ;
- les figures 6A à 6F représentent schématiquement différentes vues d'un dispositif binoculaire selon un mode de réalisation préféré de l'invention.

Dans la description qui suit, on considère que le sujet est dans une configuration assise ou debout qui est telle que sa tête est droite, c'est-à-dire que le plan de Francfort relatif à la tête du sujet est sensiblement horizontal. En anatomie, le plan de Francfort est le plan de repère qui permet l'étude du crâne. Autrement appelé plan de Virchow, il passe antérieurement par le plancher de l'orbite et postérieurement au dessus du méat acoustique externe. On dit également que le sujet est dans une position orthostatique, position dans laquelle il réalise le minimum d'efforts.

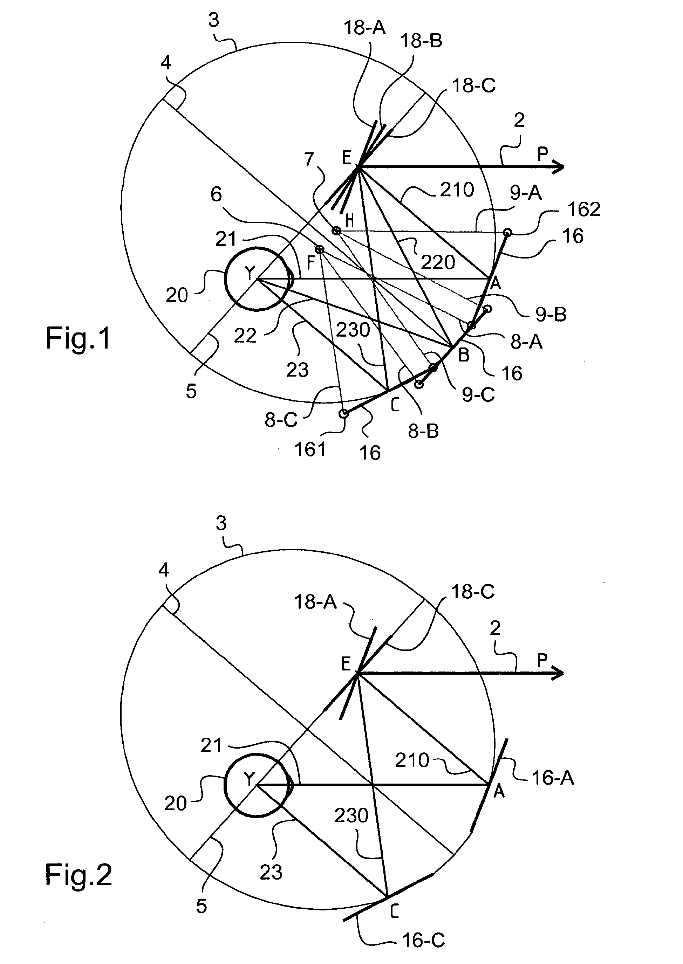

On définit un plan médian ou sagittal PSAG de la tête du sujet 30 comme étant un plan vertical parallèle à un axe antéro-postérieur de la tête et passant par un point situé à mi-distance entre les deux yeux. Le plan sagittal est parallèle au plan de la figure 1. On définit l'axe du regard ou droite de visée DV du sujet située dans un plan parallèle au plan sagittal du sujet. Dans le cas où le sujet regarde l'horizon droit devant lui à l'infini, la droite de visée est une droite horizontale DVI correspondant à l'axe de regard primaire. L'axe de regard du sujet est horizontal en position de vision de loin. Au cours de la mesure décrite plus loin, le sujet est amené à abaisser ou à élever son regard, et/ou à diriger son regard vers la droite ou vers la gauche (dans ce cas l'axe du regard n'est plus parallèle au plan PSAG), tout en maintenant sa tête 30 dans la position initiale orthostatique. Dans le cas où le sujet abaisse simplement le regard, sans aucune convergence, la droite de visée DV est une droite située dans un plan parallèle au plan sagittal et inclinée par rapport à une ligne horizontale. On définit l'axe oculaire droit comme étant l'axe passant par l'objet fixé par le sujet et le centre de la pupille de sortie (ie l'image de la pupille réelle par la cornée) de l'oeil droit. D'autres définitions sont possibles, par exemple, on peut prendre l'axe oculaire droit comme étant une droite passant par le centre de rotation de l'oeil droit et par le centre de la pupille de l'oeil droit ou encore l'axe reliant l'objet fixé à son image correspondant sur la rétine. Toutes ces définitions donnent approximativement le même axe. De même, on définit l'axe oculaire gauche comme étant l'axe passant par l'objet fixé par le sujet et le centre de la pupille de sortie de l'oeil gauche.

La position dite « vision de loin » correspond à la vision d'un objet situé à l'infini face au sujet, la droite de visée étant horizontale. L'image de l'objet étant à l'infini, l'angle de convergence des deux yeux est nul (les axes oculaires droit et gauche sont parallèles). La vision de loin est donc associée à des paramètres de proximité nulle (0 dioptries) et d'angle d'abaissement du regard nul. Il résulte de la proximité que l'angle de convergence effectif est généralement nul en vision de loin. La position dite vision de près correspond à la vision de l'image d'un objet située à une distance proche (de 20 à 40 cm par exemple) face au sujet, la droite de visée étant abaissée. En vision de près, les deux yeux convergent vers l'image de l'objet. La vision de près est donc associée à des paramètres de proximité non nulle (0.5 à 5 dioptries) et d'angle d'abaissement du regard non nul (compris entre 15 et 60 degrés). Une position de vision intermédiaire (VI) en termes de proximité (0.5 D) et d'angle d'abaissement du regard (angle d'abaissement de 15 degrés) correspond par exemple à la distance confortable pour la lecture sur un écran d'ordinateur.

La compensation optimale d'une lentille compensatrice multifocale ou progressive varie non seulement en fonction de la proximité d'une cible mais varie conjointement en fonction de l'abaissement du regard. Des études de suivi de la cinématique des yeux d'un sujet en fonction de l'abaissement du regard ont permis d'analyser le mouvement des yeux lorsqu'un sujet passe d'une position naturelle en vision de loin, avec l'axe du regard horizontal, à une position en vision de près, avec l'axe du regard abaissé, par exemple pour la lecture d'un document papier. On observe non seulement une convergence des deux yeux, qui se traduit par une modification de la distance inter-pupillaire, mais aussi de façon non limitative une rotation de chaque oeil autour son axe oculaire, une augmentation de la pression de la paupière inférieure sur la cornée, le décentrement du cristallin avec l'accommodation. Il en découle que l'orientation de l'axe physiologique et la valeur d'astigmatisme d'un oeil varie en passant d'une position naturelle de vision de loin et à la vision de près. Or, cette variation de l'astigmatisme entre la vision de près et la vision de loin n'est généralement pas prise en compte pour paramétrer une lentille compensatrice multi-focale ou une lentille compensatrice progressive. Plus généralement, il est souhaitable de mesurer précisément les paramètres de compensation oculaire (sphère, cylindre, axe, aberrations d'ordres supérieurs, kératométrie, topographie cornéenne...) en fonction de la proximité de la cible et en fonction de l'abaissement du regard pour compenser la vision en fonction de la position naturelle de l'oeil.

Nous allons maintenant détailler différents modes de réalisation du dispositif de l'invention qui permettent d'effectuer des mesures ophtalmologiques monoculaires ou binoculaires dans une pluralité de directions de visée monoculaire ou binoculaire du sujet, en particulier pour permettre une mesure en vision de loin et une mesure en vision à une distance plus proche (entre autres en vision de près et/ou en vision intermédiaire).

### Dispositif

Sur la figure 1, on a représenté en vue de côté un dispositif monoculaire ou binoculaire d'optométrie suivant une pluralité de directions de visée selon un premier mode de réalisation de l'invention. Le dispositif d'optométrie comprend un système de mesure externe non représenté solidaire du dispositif, par exemple de réflexion et/ou de réfraction oculaire et une cible de proximité variable pour stimuler l'accommodation et/ou la convergence du sujet suivant une direction de visée monoculaire ou binoculaire. Le système de mesure émet un faisceau lumineux suivant un axe optique de mesure 2 destiné à être dirigé vers l'oeil 20 du sujet à mesurer. Le système de mesure collecte le faisceau lumineux provenant de la réfraction et/ou de la réflexion par l'oeil considéré suivant le même axe optique de mesure 2. Une cible ou mire de stimulus émet un faisceau lumineux destiné à être dirigé vers l'oeil 20 du sujet à mesurer superposé à l'axe de visée de l'oeil considéré.

Sur la figure 1, on a représenté une ellipse 3 ayant un petit axe 4 et un grand axe 5, un premier foyer Y et un deuxième foyer E. Idéalement, le centre de rotation optique CRO de l'oeil 20 à mesurer est confondu le premier foyer Y de l'ellipse 3. En pratique, le dispositif comprend des moyens de réglage pour amener le premier foyer Y au voisinage du CRO de l'oeil droit 20. Par exemple, le dispositif comprend un support de tête, comprenant une mentonnière et un appui frontal permettant de maintenir la tête dans une position déterminée, et des moyens de réglage de la distance relative entre le support de tête et le premier miroir 16. Préférentiellement, la tête est en appui sur la mentonnière, et la distance entre le premier foyer Y et le centre de rotation optique de l'oeil 20 est ajustée via le champ d'observation du système de mesure pour que la pupille de l'oeil soit toujours observée selon toutes les directions de visée considérées pour la mesure. Le CRO de l'oeil doit être situé de façon à ce que l'image de la pupille par le système optique constitué des deux miroirs 16 et 18 ne soit pas décalée de plus de 10mm par rapport à l'axe de mesure 2 pour toutes les directions de visée. Ainsi l'image de la pupille reste suffisamment nette pour que la mesure puisse être réalisée suivant les directions de visée souhaitées compte tenu de la profondeur de champ du système de mesure.

Le deuxième foyer de l'ellipse 3 est placé en un point E sur l'axe optique de mesure 2 de l'appareil de mesure ophtalmologique. Le dispositif de mesure comporte en outre un système optique disposé entre l'oeil 20 du sujet et l'axe optique de mesure 2. Dans le mode de réalisation de la figure 1, le système optique est un système optique à miroirs, composé d'un premier miroir 16 et d'un deuxième miroir 18. Le premier miroir plan 16 renvoie la direction de visée vers le premier foyer E de l'ellipse 3 et le deuxième miroir plan 18 redresse l'image de l'axe de visée par le premier miroir pour l'aligner avec l'axe optique de l'appareil de mesure pour plusieurs directions de visée du sujet. À cet effet, le miroir plan 16 est tangent à l'ellipse 3. Dans ce premier mode de réalisation, le premier miroir plan 16 et le deuxième miroir plan 18 sont mobiles en translation et/ou en rotation, en fonction de la direction de visée du sujet. Sur la figure 1, on a représenté en projection dans le plan sagittal l'axe oculaire de l'oeil droit 20 d'un sujet dans trois positions : en vision de loin l'axe oculaire droit horizontal est représenté par un segment de droite 21, en vision de près l'axe oculaire droit incliné d'environ 40 degrés est représenté par un segment de droite 23 et en vision intermédiaire l'axe oculaire droit incliné d'environ 20 degrés est représenté par un segment de droite 22. On a représenté les trois chemins optiques entre le point Y et le point E correspondant respectivement à l'axe oculaire en vision de loin, en vision intermédiaire et en vision de près. Un faisceau optique se propageant suivant le segment 21 et incident sur le premier miroir 16 en un point A est réfléchi par le miroir 16 suivant l'axe 220 en direction du point E. De manière analogue, un faisceau optique se propageant suivant le segment 22, respectivement 23, et incident sur le premier miroir 16 en un point B, respectivement C, est réfléchi par le miroir 16 suivant l'axe 220, respectivement 230, en direction du point E. Le deuxième miroir 18 plan est disposé sur le trajet optique de l'appareil de mesure, l'axe optique de mesure 2 étant incident sur le deuxième miroir plan 18 au point E. Avantageusement, le deuxième miroir 18 est mobile en rotation autour du point E. On a représenté sur la figure 1 le deuxième miroir 18 selon trois orientations 18-A, 18-B et 18-C. Les orientations 18-A, 18-B et 18-C sont choisies de telle manière qu'un faisceau se propageant suivant la direction 210, respectivement 220 ou 230 soit réfléchi sur le miroir 18 selon l'orientation 18-A, respectivement 18-B ou 18-C et se propage en direction de l'appareil de mesure suivant l'axe de mesure 2. Réciproquement, un faisceau d'éclairage provenant de l'appareil de mesure et se propageant suivant l'axe de mesure 2 est incident sur le deuxième miroir 18 au point E. Suivant l'orientation 18-A, respectivement 18-B ou 18-C du deuxième miroir 18, le faisceau d'éclairage est réfléchi dans une direction 210, respectivement 220 ou 230. Le système optique constitué du premier miroir 16 et du second miroir 18 permet d'aligner optiquement l'axe de mesure 2 passant par le point E et l'axe de visée passant par le point Y et inversement, pour plusieurs directions de visée 21, 22, 23. Par conséquent, le faisceau d'éclairage provenant du point E et suivant l'axe optique 210, respectivement 220 ou 230 est réfléchi dans la direction 21 respectivement 22 ou 23 de l'axe oculaire. Le système optique formé par le premier miroir 16 mobile et le deuxième miroir 18 orientable permet d'assurer l'alignement optique entre l'axe optique de mesure 2 d'un appareil fixe et l'axe de visée du sujet, pour une pluralité de directions de visée. Dans une première position de mesure en VL, le premier miroir 16 est tangent à l'ellipse 3 en un point A et le deuxième miroir a une orientation 18-A. Dans une deuxième position de mesure en VI, le premier miroir 16 est tangent à l'ellipse 3 en un point B et le deuxième miroir a une orientation 18-B. Dans une troisième position de mesure en VP, le premier miroir 16 est tangent à l'ellipse 3 en un point C et le deuxième miroir a une orientation 18-C. Dans la position 18-A, la normale au deuxième miroir 18 est alignée sur la bissectrice entre l'axe optique de mesure 2 et l'axe 210. Dans la position 18-B, la normale au deuxième miroir 18 est alignée sur la bissectrice entre l'axe optique de mesure 2 et l'axe 220. Dans la position 18-C, la normale au deuxième miroir 18 est alignée sur la bissectrice entre l'axe optique de mesure 2 et l'axe 230. Les points A, B, et C étant tangents à l'ellipse 3 dont les foyers sont le point Y et le point E, le système permet de conserver un chemin optique identique YAE, YBE et YCE pour les directions de visée 21, 22 et 23.

Alternativement, le premier miroir 16 est un miroir concave, sphérique, elliptique ou ellipsoïdal et le deuxième miroir 18 est un miroir de puissance non nulle de telle manière que le système optique composé des miroirs 16 et 18 soit afocal au 1 er ordre des aberrations.

L'homme du métier saura trouver une came, un système de guidage, un système de biellettes articulées ou un autre système mécanique simple permettant d'effectuer un déplacement du premier miroir 16 suivant une trajectoire approchant une trajectoire elliptique.

Selon le mode de réalisation représenté sur la figure 1, le premier miroir 16 est monté mobile suivant une combinaison de translation et de rotation par un système de biellettes 8, 9. Une première biellette 8 comporte une première extrémité reliée à un point d'accrochage de biellette 6 et une deuxième extrémité reliée au miroir 16 en un point d'accrochage de biellette 161. Une deuxième biellette 9 comporte une première extrémité reliée à un point d'accrochage de biellette 7 et une deuxième extrémité reliée au miroir 16 en point d'accrochage de biellette 162. Le système à biellettes 8 et 9 s'articule de manière à ce que le mouvement du premier miroir 16 soit tangent à l'ellipse 3. Le dimensionnement du système articulé à biellettes peut se faire de la façon suivante. Au moins trois positions du premier miroir 16 (correspondant respectivement aux points A, B, C) sont placées sur l'ellipse 3 (par exemple, les deux positions extrêmes A, C et une position à mi-chemin B). Le premier miroir 16 est dimensionné par son ouverture utile issue des tracés de rayons du système de mesure pour toutes les positions d'axe oculaire respectivement 21, 22 et 23. On choisit deux points d'accrochage des biellettes 161, 162 sur le premier miroir 16. Les biellettes 8, 9 occupent au moins trois positions chacune 8-A, 8-B, 8-C et respectivement 9-A, 9-B et 9-C. Pour chacun des deux points d'accrochage des bielles 161, 162, le centre du cercle passant par les trois positions successives est calculé. Les deux centres F et respectivement H correspondent aux points d'accrochage fixes des biellettes 6 respectivement 7. Ensuite, les positions des points d'accrochage des bielles 161, 162 sur le premier miroir 16 peuvent êtres optimisés afin de minimiser les erreurs d'alignement entre l'axe AE propagé vers l'oeil et l'oeil pour les positions intermédiaires. Par ailleurs certains paramètres peuvent être optimisés (taille de l'ellipse 3, ...) ou imposés par des contraintes de coût, d'encombrement ou de poids.

Alternativement, on utilise un système de déplacement du miroir 16 à came à la place d'un système articulé à biellettes.

Quel que soit le système de déplacement choisi (biellettes, came ou autre) pour effectuer un déplacement du premier miroir tangent à l'ellipse 3, la longueur du chemin optique entre les points E et Y reste constante quel que soit le chemin optique Y-A-E, Y-B-E ou Y-C-E. De plus, l'axe EP ne varie pas en fonction de la direction de l'axe de visée. Le système optique constitué des miroirs 16 et 18 permet d'aligner l'axe optique de mesure 2 avec une image de l'axe oculaire suivant différentes directions de visée du regard 21, 22 respectivement 23. Le premier mode de réalisation permet des mesures ophtalmologiques en fonction de la direction de visée sur une grande plage angulaire. Le système articulé à biellettes permet d'utiliser un premier miroir 16 de taille limitée et donc de coût relativement réduit.

Le dispositif de la figure 1 évite de déplacer un appareil de mesure ophtalmologique pour l'aligner sur l'axe oculaire du sujet. Par définition, le trajet optique entre le point Y et le point E est constant quel que soit le trajet suivi, c'est-à-dire quelle que soit la direction de visée. Le système de conjugaison optique présente l'avantage de ne pas modifier la longueur du chemin optique entre l'oeil et l'appareil de mesure quelle que soit la direction de visée monoculaire ou binoculaire. Il n'est donc pas nécessaire de modifier la netteté d'une cible pour les différentes positions de mesure et la mise au point sur l'oeil.

Sur la figure 2, on a représenté en vue de côté un dispositif monoculaire ou binoculaire d'optométrie selon une variante du premier mode de réalisation de l'invention. La figure 2 est une variante simplifiée du premier mode de réalisation de la figure 1. Les mêmes éléments portent les mêmes signes de référence que sur la figure 1. Le dispositif de mesure comporte également un appareil de mesure (non représenté) ayant un axe de mesure 2 et un système optique à miroirs. Sur la figure 2, le système optique comporte un jeu de deux miroirs 16-A et 16-C et un deuxième miroir plan 18, analogues au dispositif de la figure 1. Dans cette variante, les miroirs 16-A et 16-C sont fixes respectivement dans les deux positions prédéterminées A et C. Le deuxième miroir 18 est un miroir plan orientable ayant également deux positions prédéterminées 18-A et 18-C. Le dispositif de la figure 2 permet d'effectuer des mesures suivant deux directions de visée monoculaire ou binoculaire, correspondant par exemple à une mesure en vision de loin et une mesure en vision de près. Le dispositif de la figure 2 permet notamment d'effectuer une mesure différenciée VL/VP. Dans les deux positions, les miroirs 16-A et 16-C sont tangents à l'ellipse, et les points E et Y sont optiquement conjugués. Le miroir 18 est mobile en rotation autour du point E, entre la première position 18-A et la deuxième position 18-C. Ces deux positions sont prédéterminées. Le système d'entraînement en rotation du deuxième miroir peut être simplifié par un système commutant entre deux butées. Selon ce second mode de réalisation, le miroir 16-A est tangent à l'ellipse 3 au point A d'intersection avec la droite de visée 21, et le miroir 16-C est tangent à l'ellipse 3 au point C d'intersection avec la droite de visée 23. L'image de la droite de visée 21 formée par le miroir 16-A passe par le point E. De même, l'image de la droite de visée 23 formée par le miroir 16-C passe par le point E. Ainsi, la longueur du chemin optique Y-A-E lorsque l'axe de visée est orienté suivant l'axe 21 et identique à la longueur du chemin optique Y-C-E lorsque l'axe de visée est orienté suivant l'axe 23. D'autre part, le miroir 18 est orienté dans la position 18-A de manière à ce que l'image de la droite de visée 21 formée par le miroir 16-A et le miroir 18 dans la position 18-A soit superposé avec l'axe de mesure 2. De même, le miroir 18 est orienté dans la position 18-C de manière à ce que l'image de la droite de visée 23 formée par le miroir 16-C et le miroir 18 dans la position 18-C soit superposé avec l'axe de mesure 2.

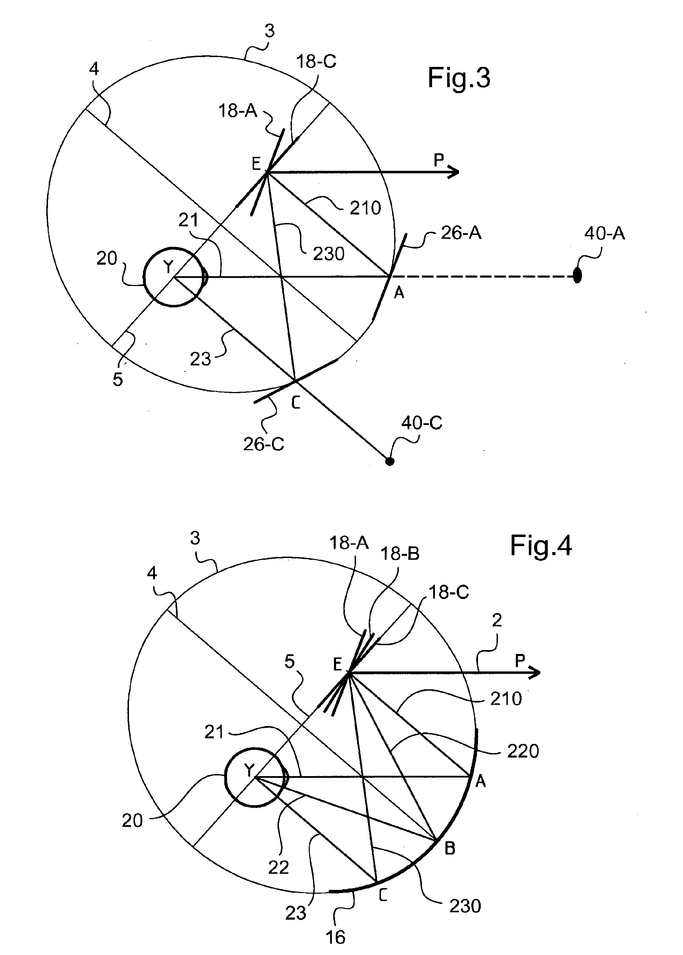

Sur la figure 3, on a représenté une deuxième variante du mode de réalisation de la figure 2. Le dispositif de la figure 3 comporte deux mires 40-A et 40-C de proximités différentes qui sont séparées de l'appareil de mesure ophtalmologique. À titre d'exemple illustratif et non-limitatif, la mire 40-A a une proximité correspondant à une vision de loin, et la mire 40-A a une proximité correspondant à une vision de près. Dans cette deuxième variante, les miroirs 16-A et 16-C sont remplacés par des lames dichroïques 26-A respectivement 26-C, dites lames chaudes. Les lames chaudes 26-A et 26-C sont aptes à transmettre un faisceau visible (400-700 nm) et à réfléchir un faisceau dans le proche infrarouge (750-1100nm). On définit une ellipse 3 ayant pour foyers un point Y et un point E. Le point Y est destiné à être superposé avec le CRO de l'oeil mesuré. La lame chaude 26-A est tangente à l'ellipse 3 au point A, et la lame chaude 26-C est tangente à l'ellipse 3 au point C. Le miroir 18 coupe l'axe optique de mesure 2 au point E et pivote autour du point E entre une position 18-A et une position 18-C.

Dans une première position de mesure, la mire 40-A émet un faisceau optique de stimulation dans le visible de manière à stimuler l'accommodation du sujet en vision de loin, tandis que la mire 40-C est éteinte. Le miroir 18 est alors dans la position 18-A. La lame chaude 26-A transmet le faisceau optique de stimulation visible de manière à superposer l'axe optique du faisceau de stimulation avec la direction de visée 21 qui passe par le point A et par le point Y. L'appareil de mesure génère un faisceau d'éclairage dans le proche infrarouge aligné avec l'axe optique de mesure 2. Le miroir 18 en position 18-A réfléchit le faisceau d'éclairage dans la direction 210 passant par le point E et le point A. La lame chaude 26-A réfléchit le faisceau d'éclairage proche infrarouge dans la direction de l'axe de visée 21, correspond à une direction de visée en vision de loin. Le faisceau de mesure est formé par réflexion et/ou réfraction du faisceau d'éclairage par l'oeil 20 dans la direction de visée 21. La lame chaude 26-A réfléchit le faisceau de mesure dans la direction 210 vers le point E sur le miroir 18. Le miroir 18 en position 18-A réfléchit le faisceau de mesure dans l'axe de mesure 2. Le dispositif permet ainsi d'effectuer une mesure ophtalmologique l'oeil étant orienté suivant une première direction de visée 21.

Dans une autre position de mesure, la mire 40-C émet un faisceau optique de stimulation dans le visible de manière à stimuler l'accommodation du sujet en vision de près, tandis que la mire 40-A est éteinte. Le miroir 18 est alors dans la position 18-C. La lame chaude 26-C transmet le faisceau optique de stimulation visible de manière à superposer l'axe optique du faisceau de stimulation avec une autre direction de visée 23 qui passe par le point C et par le point Y. L'appareil de mesure génère un faisceau d'éclairage dans le proche infrarouge aligné avec l'axe optique de mesure 2. Le miroir 18 en position 18-C réfléchit le faisceau d'éclairage dans la direction 230 passant par le point E et le point C. La lame chaude 26-C réfléchit le faisceau d'éclairage proche infrarouge dans la direction de l'axe de visée 23, correspond à une direction de visée en vision de près. Le faisceau de mesure est formé par réflexion et/ou réfraction du faisceau d'éclairage par l'oeil 20 dans la direction de visée 23. La lame chaude 26-C réfléchit le faisceau de mesure dans la direction 230 vers le point E sur le miroir 18. Le miroir 18 en position 18-C réfléchit le faisceau de mesure dans l'axe de mesure 2.

Selon la deuxième variante du premier mode de réalisation, illustrée par la figure 3, le système optique formé par les lames chaudes 26-A, 26-C et le miroir pivotant 18 permet d'aligner l'axe optique de mesure 2 avec une direction de visée 21 en vision de loin et avec une direction de visée 23 en vision de près, suivant deux chemins optiques, respectivement E-A-Y et E-C-Y, ayant une même longueur optique.

Sur la figure 4, on a représenté en vue de côté un dispositif d'optométrie en fonction de la direction de visée selon un deuxième mode de réalisation de l'invention. Le dispositif de la figure 4 comporte un système optique formé un premier miroir 16 et un deuxième miroir 18. Avantageusement, le premier miroir 16 est formé d'une portion de l'ellipse 3 et a pour foyers optiques les points E et Y. Le deuxième miroir 18 de renvoi est un miroir de puissance non nulle ou un miroir déformable apte à corriger les aberrations optiques du premier miroir 16. Le deuxième miroir 18 est monté pivotant autour du point E. Contrairement au premier mode de réalisation, le premier miroir ellipsoïdal 16 reste fixe. Par définition, les points E et Y sont optiquement conjugués à travers le premier miroir 16. On a représenté sur la figure 4, trois positions de mesure, correspondant à trois directions de visée monoculaire ou binoculaire. Un faisceau optique se propageant suivant le segment 21 et incident sur le premier miroir 16 en un point A est réfléchi par le miroir 16 suivant l'axe 210 en direction du point E. De même, un faisceau optique se propageant suivant le segment 22, respectivement 23, et incident sur le premier miroir 16 en un point B, respectivement C, est réfléchi par le miroir 16 suivant l'axe 220, respectivement 230, en direction du point E. Le deuxième miroir 18 est orientable suivant au moins trois positions 18-A, respectivement 18-B et 18-C de manière à redresser l'image de l'axe oculaire à travers le premier miroir 16 pour l'aligner sur l'axe optique de mesure 2. Dans la position 18-A, la normale au deuxième miroir 18 est alignée sur la bissectrice entre l'axe optique de mesure 2 et l'axe 210. Dans la position 18-B, la normale au deuxième miroir 18 est alignée sur la bissectrice entre l'axe optique de mesure 2 et l'axe 220. Dans la position 18-C, la normale au deuxième miroir 18 est alignée sur la bissectrice entre l'axe optique de mesure 2 et l'axe 230. Avantageusement, le deuxième miroir 18 est un miroir déformable pour compenser les aberrations du premier miroir 16 en fonction de la direction de visée monoculaire ou binoculaire, c'est-à-dire en fonction de l'orientation du miroir 18. La déformation compensatrice appliquée au deuxième miroir 18 peut être prédéfinie en fonction de l'orientation du deuxième miroir 18. Le premier miroir 16 ellipsoïdal permet de conjuguer optiquement le centre de rotation optique de l'oeil confondu avec le point Y avec le point E sur l'axe optique de mesure 2 avec une longueur de chemin optique constante pour différentes directions de visée monoculaire ou binoculaire. Le deuxième miroir 18 permet de redresser l'image de l'axe oculaire et de l'aligner sur l'axe optique de mesure 2. Le système de conjugaison optique de la figure 4 illustre le fonctionnement pour trois positions de mesure. Toutefois, le premier miroir 16 ellipsoïdal permet une mesure sur une plage continue de directions de visée monoculaire pu binoculaire du regard. D'autres mesures sont possibles pour d'autres directions de visée monoculaire ou binoculaire. Il suffit d'orienter le deuxième miroir 18 en fonction de la direction de visée monoculaire ou binoculaire, de manière à ce que l'axe optique de mesure 2 soit aligné avec l'image de l'axe oculaire pour une direction de visée monoculaire ou binoculaire particulière à travers le système de conjugaison optique. L'avantage du dispositif de la figure 4 est de nécessiter le déplacement d'un seul composant, le second miroir 18, le premier miroir 16 restant fixe. Dans ce mode de réalisation, un seul mouvement de rotation du deuxième miroir plan 18 suffit pour aligner l'axe optique de mesure 2 avec l'image de l'axe oculaire selon différentes directions de visée. Toutefois, la gamme de mesure, en fonction de la direction de visée monoculaire ou binoculaire, est liée à l'étendue du miroir ellipsoïdal. Plus la gamme de mesure angulaire souhaitée est étendue et plus le coût du miroir ellipsoïdal est élevé, et plus les aberrations sont difficiles à corriger.

Le dispositif décrit en lien avec les figures 1 à 4 correspond à une mesure monoculaire ou binoculaire suivant une pluralité de directions de visée.

Le plan des figures 1 à 4 contenant l'axe 5 de l'ellipse 3 peut être parallèle au plan sagittal dans le cas où la variation de direction du regard correspond à un abaissement du regard sans mouvement de convergence ; alternativement le plan des figures 1 à 4 contenant l'axe 5 de l'ellipse 3 peut être parallèle au plan de Francfort dans le cas où la variation de direction du regard correspond à une convergence binoculaire sans variation de l'abaissement du regard. Dans le cas d'une combinaison d'un mouvement d'abaissement et de convergence du regard, le plan des figures 1 à 4 peut être un plan incliné d'une inclinaison fixe par rapport au plan sagittal. Selon un mode de réalisation avantageux, le dispositif comporte des moyens de rotation, aptes à faire tourner le plan contenant l'axe 5 de l'ellipse 3 autour de l'axe 5. Selon encore un autre mode de réalisation, le plan de l'ellipse 3 peut être incliné autour de l'axe YA, l'abaissement et la convergence étant liés de manière prédéterminée.

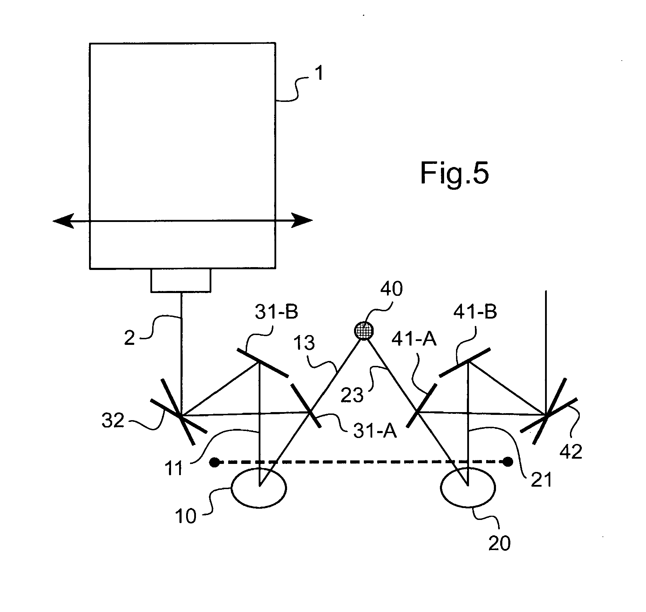

Sur la figure 5, on a représenté un système de mesure ophtalmologique selon un troisième mode de réalisation de l'invention. Le dispositif comporte un appareil de mesure monoculaire 1 apte à effectuer une mesure monoculaire suivant un axe optique de mesure 2. Préférentiellement, l'appareil de mesure 1 comporte une cible interne apte à générer un faisceau optique de stimulation en vision de loin. L'appareil de mesure monoculaire 1 est mobile en translation (flèche horizontale) pour pouvoir être disposé face à l'oeil à mesurer. Le dispositif de la figure 5 comprend une cible 40 binoculaire apte à stimuler l'accommodation et la convergence des deux yeux simultanément. Avantageusement, la cible 40 a une proximité correspondant à une vision de près. Le dispositif comporte également un système d'alignement optique pour chaque oeil, de manière à effectuer des mesures suivant plusieurs directions de visée du regard sans modifier la longueur du chemin optique. Un système optique comprenant deux miroirs chauds 31-A, 41-A est situé au plus proche des yeux pour permettre de stimuler la convergence et l'accommodation en VP. Le point de fixation 40 est situé à une proximité entre 0.5 et 10 dioptries dans le plan sagittal afin de solliciter la convergence oculaire. Avantageusement, les miroirs chauds 31-A, 41-A et la cible 40 sont disposés de manière à combiner un abaissement et une convergence du regard binoculaire en VP. Les miroirs chauds 31-A et 41-A permettent d'observer le point de fixation 40 dans le visible, tout en renvoyant le faisceau de mesure infrarouge vers un miroir pivotant 32, respectivement 42, en direction de l'axe de mesure 2 de l'appareil de mesure monoculaire 1. Lorsque la cible 40 en VP est allumée, le dispositif permet ainsi une mesure monoculaire dans une direction de visée en VP, respectivement 13 pour l'oeil gauche 10 et 23 pour l'oeil droit 20. Le dispositif comporte en outre des miroirs chauds 31-B, 41-B disposés de manière à permettre une mesure en VL dans une direction de visée, respectivement 11 pour l'oeil gauche 10 et 21 pour l'oeil droit 20. Les miroirs orientables 32, 42 permettent de passer d'une position de mesure en VL à une autre position de mesure en VP, et inversement. En position VL l'oeil voit le stimulus de l'appareil de mesure 1 qui est donc monoculaire. Alors qu'en position VP, le stimulus 40 est vu par les deux yeux permettant d'abaisser le regard, de converger et d'accommoder vers un unique point. L'appareil de mesure monoculaire permet ainsi de réaliser une mesure en VL et en VP. Le miroir 31-A, respectivement le miroir 31-B, est disposé de manière à être tangent en un point A, respectivement en un point B, à une ellipse ayant pour foyers un point Y aligné sur le CRO de l'oeil gauche 10 et un point E situé à l'intersection de l'axe de mesure 2 et du miroir 32. De même, le miroir 41-A, respectivement le miroir 41-B, est disposé de manière à être tangent en un point A', respectivement en un point B', à une ellipse ayant pour foyers un point Y' aligné sur le CRO de l'oeil droit 20 et un point E' situé à l'intersection de l'axe de mesure 2 et du miroir 42. De cette manière, le chemin optique entre le CRO de l'oeil mesuré et l'appareil de mesure ophtalmologique 1 est identique dans la direction de visée en VP et en VL.

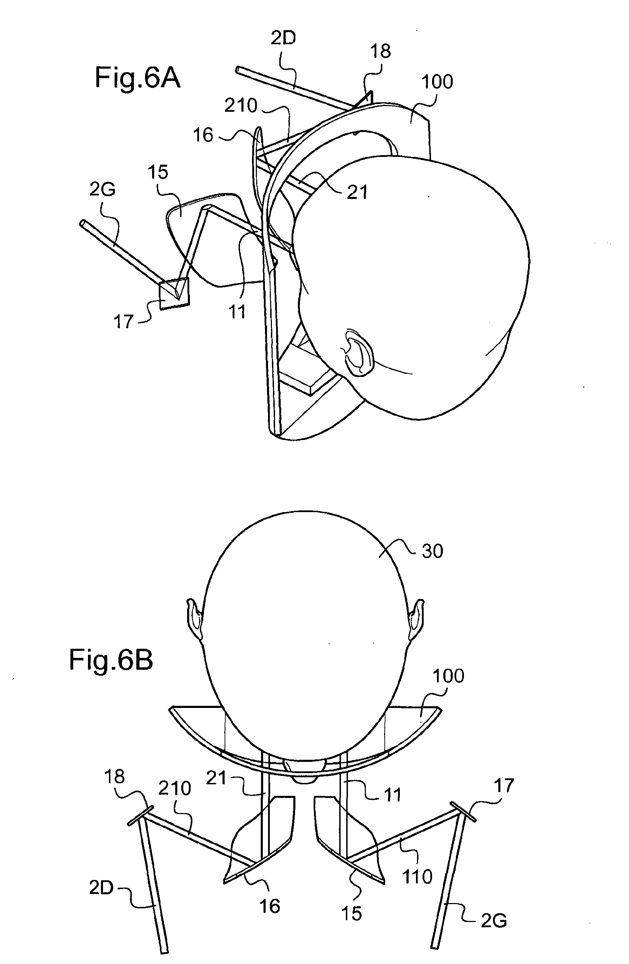

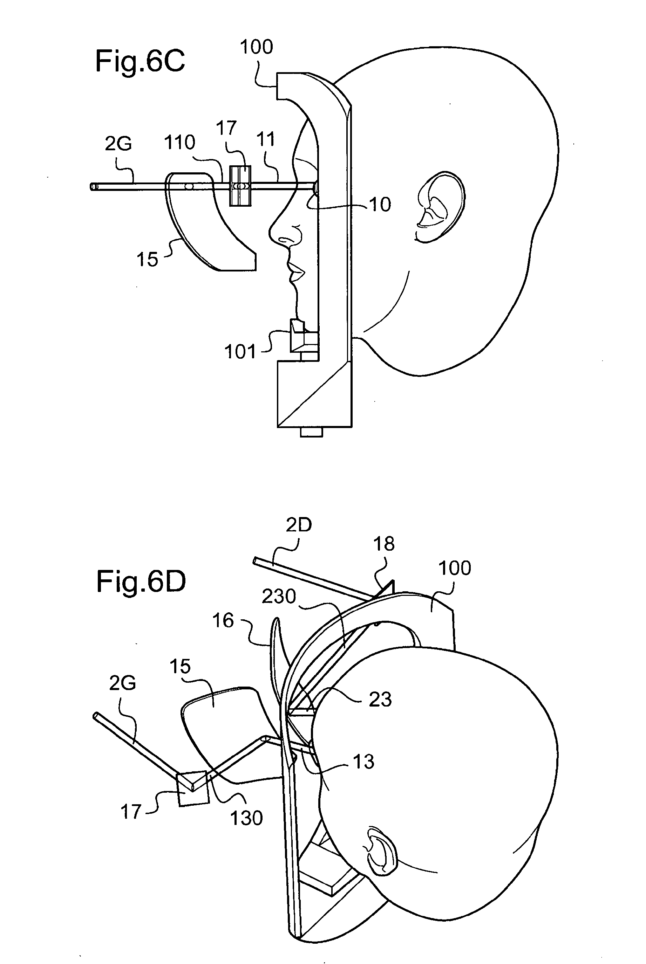

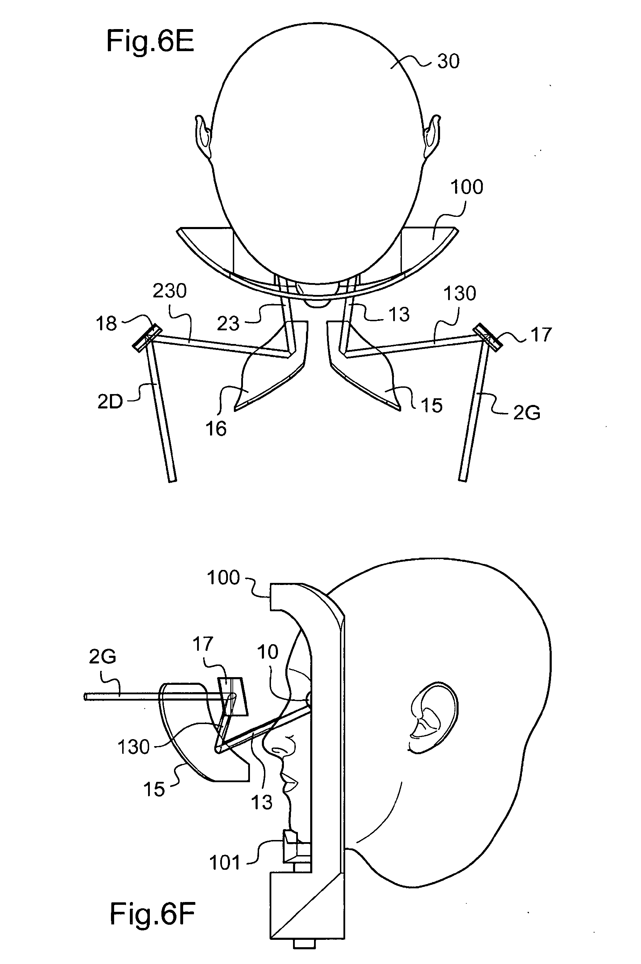

Les figures 6A-6F illustrent schématiquement différentes vues d'un dispositif binoculaire selon un mode de réalisation préféré de l'invention. Il s'agit d'une variante du dispositif de la figure 5, dans laquelle un appareil de mesure monoculaire mobile en translation est remplacé par deux appareils de mesure monoculaire ayant respectivement un axe de mesure droit 2D et un axe de mesure gauche 2G, dédiés respectivement à l'oeil droit et à l'oeil gauche, afin d'éviter le mouvement de translation. Les figures 6A, 6B et 6C illustrent le fonctionnement du dispositif en VL, la droite de visée étant horizontale. Les figures 6D, 6E et 6F illustrent le fonctionnement du dispositif en VP, la droite de visée étant abaissée par rapport à l'horizontale et le regard étant convergent. Les figures 6A et 6D sont des vues en perspective du dispositif de mesure, les figures 6B et 6E des vues de dessus et les figures 6B et 6E des vues de face du dispositif. Les mêmes signes de référence correspondent aux mêmes éléments que décrits en lien avec les autres modes de réalisation. On n'a représenté sur les figures 6A-6F uniquement le système d'alignement optique, les autres éléments n'étant pas représentés. Le dispositif comporte une cible binoculaire apte à stimuler l'accommodation et la convergence des deux yeux simultanément. Sur les figures 6A et 6D, on observe au premier plan l'axe de mesure 2G de l'oeil gauche du sujet et au second plan l'axe de mesure 2D de l'oeil droit du sujet. Le dispositif comporte un support de tête 100 comprenant une mentonnière 101 et une zone d'appui frontal pour maintenir la tête 30 du sujet dans une position fixe. Avantageusement, la tête du sujet est maintenue dans une position où le plan de Francfort est horizontal. Pour certaines mesures particulières, le support de tête peut être incliné de manière à effectuer des mesures dans d'autres postures de tête du sujet. Le dispositif comporte un premier système optique comprenant un premier miroir 16 ellipsoïdal fixe, un deuxième miroir 18 pivotant ou mobile en rotation, et un appareil de mesure ophtalmologique dédié à l'oeil droit 20 suivant l'axe de mesure 2D. Le dispositif comporte également un deuxième système d'alignement optique comprenant un premier miroir 15 ellipsoïdal fixe (et non un miroir mobile suivant une trajectoire elliptique), un deuxième miroir 17 de puissance non nulle, pivotant ou mobile en rotation, et un appareil de mesure ophtalmologique dédié à l'oeil gauche 10 suivant l'axe de mesure 2G. Le dispositif des figures 6A-6F comporte également des moyens de réglage agissant sur la partie 101 de la mentonnière 100 et/ou sur le système d'alignement optique, pour amener un foyer du miroir ellipsoïdal 15 au voisinage du centre de rotation optique de l'oeil gauche 10. De même, des moyens de réglage sont prévus pour amener un foyer du miroir ellipsoïdal 16 au voisinage du CRO de l'oeil droit 20. Les réglages en hauteur des systèmes d'alignement optique droit et gauche peuvent être différents pour aligner les axes de mesures en fonction de la hauteur des yeux du sujet. Avantageusement, les systèmes d'alignement optique droit et gauche sont couplés, en particulier l'orientation des miroirs 17 et 18.

Sur les figures 6A-6C, le sujet observe une mire dans une posture de vision correspondant à la vision de loin : l'axe oculaire droit 21 et l'axe oculaire gauche 11 sont horizontaux et sensiblement parallèles entre eux (aux défauts de vision du sujet près). Les miroirs ellipsoïdaux 15 et respectivement 16 renvoient l'axe oculaire gauche 11 suivant une direction 110, respectivement l'axe oculaire droit 21 suivant une direction 210, vers le second miroir 17, respectivement 18. Dans cette posture de vision de loin, le miroir 17 est orienté de manière à aligner l'image de l'axe oculaire gauche sur l'axe de mesure gauche 2G, et respectivement le miroir 18 est orienté de manière à aligner l'image de l'axe oculaire droit sur l'axe de mesure droit 2D.

Sur les figures 6D-6F, le sujet observe une mire dans une posture de vision correspondant à la vision de près : l'axe oculaire droit 23 et l'axe oculaire gauche 13 sont abaissés et convergent. Les miroirs ellipsoïdaux 15 et respectivement 16 renvoient l'axe oculaire gauche 13 suivant une direction 130, respectivement l'axe oculaire droit 23 suivant une direction 230, vers le second miroir 17, respectivement 18. Les miroirs ellipsoïdaux 15, 16 restent fixes dans les différentes postures de mesure. Dans la posture de vision de près, le miroir 17 est orienté de manière à aligner l'image de l'axe oculaire gauche 230 sur l'axe de mesure gauche 2G, et respectivement le miroir 18 est orienté de manière à aligner l'image de l'axe oculaire droit 130 sur l'axe de mesure droit 2D.

Le dispositif des figures 6A-6F permet d'effectuer une mesure monoculaire de chaque oeil suivant une pluralité de directions de visée binoculaires. Le système d'alignement optique comprenant les miroirs ellipsoïdaux 15 et 16, et les miroirs 17, 18 permet d'effectuer une mesure ophtalmologique suivant une pluralité de directions de visée du regard, tout en assurant la conservation de la longueur du chemin optique suivant les différentes directions de visée du regard. Les appareils de mesure monoculaire gauche et droit restent fixes. Avantageusement, seuls les seconds miroirs 17 et 18 sont mobiles en rotation autour d'un point de pivot et compensent aux 1^{er} ordres les aberrations générées par les ellipsoïdaux 15 et 16.

La valeur du paramètre de demi-écart pupillaire (1/2 IPD) peut être adaptée de façon symétrique ou non (gérée par demi-IPD) et à partir d'une mesure extérieure (Visioffice ou pupillomètre) ou à partir d'un retour du réglage de l'appareil en VL lors de l'alignement.

L'invention est particulièrement adaptée pour toute personne qui pratique des mesures ophtalmologiques par réfraction et qui désire proposer ou effectuer des mesures en suivant l'inclinaison physiologique des yeux du sujet.

Le dispositif de l'invention peut être utilisé par un optométriste ou un ophtalmologiste, ou encore par un opticien pour déterminer les paramètres de personnalisation d'un verre de lunettes.

Le dispositif de l'invention peut servir à définir les moyens nécessaires à la prescription d'une lentille compensatrice multi-focale ou progressive.

L'invention permet d'adapter un appareil de mesure ophtalmologique monoculaire ou binoculaire ayant un axe optique de mesure pour chaque oeil, pour permettre des mesures suivant une pluralité de directions de visée monoculaires ou binoculaires. Avantageusement, le dispositif de l'invention permet une mesure ophtalmologique différenciée en vision de loin et en vision de près tenant compte de la direction de visée du regard.

## Revendications

1. Dispositif de détermination d'au moins un paramètre de vision d'un sujet suivant une pluralité de directions de visée monoculaire ou binoculaire du sujet, ledit dispositif comprenant :
- un appareil de mesure ophtalmologique monoculaire ou binoculaire, comprenant des moyens de mesure ophtalmologique du paramètre de vision à déterminer aptes à émettre un faisceau optique d'éclairage et à recevoir un faisceau optique de mesure suivant au moins un axe optique de mesure (2) aligné avec une direction prédéterminée de visée de l'oeil concerné,
- des moyens de stimulation visuelle aptes à générer un faisceau optique de stimulation suivant un axe optique de stimulation aligné avec ladite direction prédéterminée de visée de l'oeil concerné, et
- des moyens de support de tête aptes à recevoir la tête d'un sujet et à la maintenir dans une posture déterminée ;
**caractérisé en ce que** :
étant définie une ellipse (3) ayant un premier foyer (E) sur ledit axe optique de mesure (2) et un deuxième foyer (Y), ledit dispositif comprend :
- au moins un système d'alignement optique destiné à être disposé entre les moyens de mesure ophtalmologique (1) et l'oeil concerné (10, 20), ledit système d'alignement optique étant apte à réfléchir lesdits faisceaux optiques d'éclairage et de mesure entre les foyers (E, Y) de l'ellipse (3), et
- des moyens de réglage aptes à modifier la position relative dudit système d'alignement optique par rapport aux moyens de support de tête de manière à amener le deuxième foyer (Y) au voisinage du centre de rotation de l'oeil concerné du sujet,
- ledit système d'alignement optique comprenant des premiers moyens optiques réfléchissants (15, 16, 16-A, 16-C, 31-A, 31-B, 41-A, 41-B) et des seconds moyens optiques réfléchissants (17, 18, 32, 42), lesdits premiers moyens optiques réfléchissants étant tangents à ladite ellipse (3) en un premier point (A) sur une première direction de visée (11, 21) et en au moins un autre point (B, C) sur au moins une autre direction de visée (12, 13, 22, 23) de l'oeil concerné, et lesdits seconds moyens optiques réfléchissants (17, 18, 32, 42) étant montés mobiles en rotation autour du premier foyer (E) entre une première position (18-A) dans laquelle le système d'alignement optique aligne ladite première direction de visée (11, 21) avec l'axe optique de mesure (2) et au moins une autre position (18-B, 18-C) dans laquelle le système d'alignement optique aligne ladite au moins une autre direction de visée (12, 13, 22, 23) de l'oeil concerné avec l'axe optique de mesure (2).

2. Dispositif de détermination d'au moins un paramètre de vision selon la revendication 1, dans lequel les premiers moyens optiques réfléchissants comportent un miroir sphérique, un miroir plan, une pluralité de miroirs plans (16-A, 16-C), une lame dichroïque (31-A, 31-B) ou une pluralité de lames dichroïques (31-A, 31-B, 41-A, 41-B).

3. Dispositif de détermination d'au moins un paramètre de vision selon la revendication 1 ou la revendication 2, dans lequel lesdits premiers moyens optiques réfléchissants comprennent un premier miroir (15, 16) et dans lequel ledit système d'alignement optique comprend des moyens de déplacement du premier miroir (15, 16) aptes à déplacer le premier miroir (15, 16) suivant une trajectoire prédéterminée en fonction de la direction de visée monoculaire ou binoculaire du sujet.

4. Dispositif de détermination d'au moins un paramètre de vision selon la revendication 3, dans lequel lesdits moyens de déplacement du premier miroir comprennent un système articulé à biellettes, une came et/ou un système mécanique de guidage.

5. Dispositif de détermination d'au moins un paramètre de vision selon l'une des revendications 3 ou 4, dans lequel ladite trajectoire prédéterminée est une trajectoire elliptique et dans lequel ledit premier miroir (15, 16) est orienté de manière à être tangent à ladite trajectoire elliptique.

6. Dispositif de détermination d'au moins un paramètre de vision selon l'une des revendications 1 à 5, dans lequel lesdits premiers moyens optiques réfléchissants comprennent un premier miroir (16-A) ayant une première position prédéterminée tangente à ladite ellipse (3) en un premier point (A) et un deuxième miroir (16-C) ayant une deuxième position prédéterminée tangente à ladite ellipse (3) en un autre point (C).

7. Dispositif de détermination d'au moins un paramètre de vision selon la revendication 1, dans lequel lesdits premiers moyens optiques réfléchissants comprennent un miroir ellipsoïdal et lesdits seconds moyens optiques réfléchissants comprennent un second miroir de puissance optique non nulle, de manière à ce que le système d'alignement optique constitué du premier et du second miroir soit afocal au premier ordre.

8. Dispositif de détermination d'au moins un paramètre de vision selon l'une des revendications 1 à 7, dans lequel lesdits seconds moyens optiques réfléchissants (17, 18, 32, 42) comportent un miroir plan.

9. Dispositif de détermination d'au moins un paramètre de vision selon l'une des revendications 1 à 8, dans lequel ledit système d'alignement comprend un moyen d'orientation des seconds moyens optiques réfléchissants (17, 18, 32, 42) apte à faire pivoter seconds moyens optiques réfléchissants (17, 18, 32, 42) autour du premier foyer (E) de l'ellipse (3) en fonction de la direction (11, 21, 12, 22, 13, 23) de visée monoculaire ou binoculaire.

10. Dispositif de détermination d'au moins un paramètre de vision selon l'une des revendications 1 à 9, comprenant des moyens d'inclinaison dudit système d'alignement optique, lesdits moyens d'inclinaison étant aptes à orienter le plan de l'ellipse (3) autour d'un axe passant par ses foyers.

11. Dispositif de détermination d'au moins un paramètre de vision selon l'une des revendications 1 à 10, dans lequel ledit système d'alignement optique comprend au moins une première position prédéterminée associée à une première direction de visée et au moins une autre position prédéterminée associée à au moins une autre direction de visée.

12. Dispositif de détermination d'au moins un paramètre de vision selon la revendication 11, dans lequel ladite première direction de visée correspond à une direction horizontale droit devant le sujet et dans lequel ladite au moins une autre direction de visée correspond à une direction de visée en vision de près, inclinée par rapport à l'horizontale.

13. Dispositif de détermination d'au moins un paramètre de vision selon l'une des revendications 1 à 12, dans lequel les moyens de mesure ophtalmologique sont aptes à mesurer et à enregistrer au moins un paramètre de vision de type sphère, cylindre, axe, aberration d'ordre supérieur, de kératométrie et/ou de topographie cornéenne, et/ou de diamètre de pupille dans une première direction de visée et dans au moins une autre direction de visée, et/ou une différence entre un paramètre de vision mesuré dans ladite première direction de visée et mesuré dans ladite au moins une autre direction de visée.

14. Dispositif de détermination d'au moins un paramètre de vision selon l'une des revendications 1 à 13, dans lequel ledit appareil de mesure ophtalmologique est un appareil binoculaire ayant un premier axe de mesure associé à l'oeil droit du sujet et un deuxième axe de mesure associé à l'oeil gauche du sujet, et dans lequel ledit système d'alignement optique consiste en un premier système d'alignement optique et un deuxième système d'alignement optique,le premier système d'alignement optique étant destiné à être disposé entre ledit appareil de mesure ophtalmologique binoculaire et l'oeil droit du sujet, et
- le deuxième système d'alignement optique étant destiné à être disposé entre ledit appareil de mesure ophtalmologique binoculaire et l'oeil gauche dudit sujet.

## Patentansprüche

1. Vorrichtung zur Bestimmung mindestens eines Sichtparameters einer Person gemäß einer Vielzahl von monokularen oder binokularen Blickrichtungen der Person, wobei die Vorrichtung enthält:
- ein monokulares oder binokulares ophthalmologisches Messgerät, das Einrichtungen zur ophthalmologischen Messung des zu bestimmenden Sichtparameters enthält, die in der Lage sind, einen optischen Beleuchtungsstrahl zu emittieren und einen optischen Messstrahl gemäß mindestens einer optischen Messachse (2) zu empfangen, die mit einer vorbestimmten Blickrichtung des betroffenen Auges ausgerichtet ist,
- Einrichtungen zur visuellen Stimulation, die in der Lage sind, einen optischen Stimulationsstrahl gemäß einer optischen Stimulationsachse zu erzeugen, die mit der vorbestimmten Blickachse des betroffenen Auges ausgerichtet ist, und
- Kopfstützeinrichtungen, die in der Lage sind, den Kopf einer Person aufzunehmen und ihn in einer bestimmten Positur zu halten;
**dadurch gekennzeichnet, dass**:
wenn eine Ellipse (3) mit einem ersten Brennpunkt (E) auf der optischen Messachse (2) und mit einem zweiten Brennpunkt (Y) definiert ist, die Vorrichtung enthält:
- mindestens ein optisches Ausrichtsystem, das dazu bestimmt ist, zwischen den ophthalmologischen Messeinrichtungen (1) und dem betroffenen Auge (10, 20) angeordnet zu sein, wobei das optische Ausrichtsystem in der Lage ist, die optischen Beleuchtungs- und Messstrahlen zwischen den Brennpunkten (E, Y) der Ellipse (3) zu reflektieren, und
- Einstelleinrichtungen, die in der Lage sind, die relative Stellung des optischen Ausrichtsystems bezüglich der Kopfstützeinrichtungen so zu verändern, dass der zweite Brennpunkt (Y) in die Nähe des Drehzentrums des betroffenen Auges der Person gebracht wird,
- wobei das optische Ausrichtsystem erste reflektierende optische Einrichtungen (15, 16, 16-A, 16-C, 31-A, 31-B, 41-A, 41-B) und zweite reflektierende optische Einrichtungen (17, 18, 32, 42) enthält, wobei die ersten reflektierenden optischen Einrichtungen die Ellipse (3) an einem ersten Punkt (A) in einer ersten Blickrichtung (11, 21) und an mindestens einem anderen Punkt (B, C) in mindestens einer anderen Blickrichtung (12, 13, 22, 23) des betroffenen Auges tangieren, und die zweiten reflektierenden optischen Einrichtungen (17, 18, 32, 42) um den ersten Brennpunkt (E) zwischen einer ersten Stellung (18-A), in der das optische Ausrichtsystem die erste Blickrichtung (11, 21) mit der optischen Messachse (2) ausrichtet, und mindestens einer anderen Stellung (18-B, 18-C) drehbeweglich montiert sind, in der das optische Ausrichtsystem die mindestens eine andere Blickrichtung (12, 13, 22, 23) des betroffenen Auges mit der optischen Messachse (2) ausrichtet.

2. Vorrichtung zur Bestimmung mindestens eines Sichtparameters nach Anspruch 1, wobei die ersten reflektierenden optischen Einrichtungen einen sphärischen Spiegel, einen ebenen Spiegel, eine Vielzahl ebener Spiegel (16-A, 16-C), ein dichroitisches Plättchen (31-A, 31-B) oder eine Vielzahl dichroitischer Plättchen (31-A, 31-B, 41-A, 41-B) aufweisen.

3. Vorrichtung zur Bestimmung mindestens eines Sichtparameters nach Anspruch 1 oder Anspruch 2, wobei die ersten reflektierenden optischen Einrichtungen einen ersten Spiegel (15, 16) enthalten, und wobei das optische Ausrichtsystem Verschiebeeinrichtungen des ersten Spiegels (15, 16) enthält, die in der Lage sind, den ersten Spiegel (15, 16) gemäß einer vorbestimmten Strecke abhängig von der monokularen oder binokularen Blickrichtung der Person zu verschieben.

4. Vorrichtung zur Bestimmung mindestens eines Sichtparameters nach Anspruch 3, wobei die Verschiebeeinrichtungen des ersten Spiegels ein gelenkiges System mit Schwingarmen, eine Nocke und/oder ein mechanisches Führungssystem enthalten.

5. Vorrichtung zur Bestimmung mindestens eines Sichtparameters nach einem der Ansprüche 3 oder 4, wobei die vorbestimmte Strecke eine elliptische Strecke ist, und wobei der erste Spiegel (15, 16) so ausgerichtet ist, dass er die elliptische Strecke tangiert.

6. Vorrichtung zur Bestimmung mindestens eines Sichtparameters nach einem der Ansprüche 1 bis 5, wobei die ersten reflektierenden optischen Einrichtungen einen ersten Spiegel (16-A), der eine die Ellipse (3) an einem ersten Punkt (A) tangierende vorbestimmte erste Stellung hat, und einen zweiten Spiegel (16-C) enthalten, der eine die Ellipse (3) an einem anderen Punkt (C) tangierende vorbestimmte zweite Stellung hat.

7. Vorrichtung zur Bestimmung mindestens eines Sichtparameters nach Anspruch 1, wobei die ersten reflektierenden optischen Einrichtungen einen ellipsenförmigen Spiegel enthalten, und die zweiten reflektierenden optischen Einrichtungen einen zweiten Spiegel mit einer optischen Leistung ungleich Null enthalten, so dass das aus dem ersten und dem zweiten Spiegel bestehende optische Ausrichtsystem in erster Ordnung afokal ist.

8. Vorrichtung zur Bestimmung mindestens eines Sichtparameters nach einem der Ansprüche 1 bis 7, wobei die zweiten reflektierenden optischen Einrichtungen (17, 18, 32, 42) einen ebenen Spiegel aufweisen.

9. Vorrichtung zur Bestimmung mindestens eines Sichtparameters nach einem der Ansprüche 1 bis 8, wobei das Ausrichtsystem eine Einrichtung zur Ausrichtung der zweiten reflektierenden optischen Einrichtungen (17, 18, 32, 42) enthält, die in der Lage ist, die zweiten reflektierenden optischen Einrichtungen (17, 18, 32, 42) abhängig von der monokularen oder binokularen Blickrichtung (11, 21, 12, 22, 13, 23) um den ersten Brennpunkt (E) der Ellipse (3) zu schwenken.

10. Vorrichtung zur Bestimmung mindestens eines Sichtparameters nach einem der Ansprüche 1 bis 9, die Neigungseinrichtungen des optischen Ausrichtsystems enthält, wobei die Neigungseinrichtungen in der Lage sind, die Ebene der Ellipse (3) um eine durch ihre Brennpunkte gehende Achse auszurichten.

11. Vorrichtung zur Bestimmung mindestens eines Sichtparameters nach einem der Ansprüche 1 bis 10, wobei das optische Ausrichtsystem mindestens eine erste vorbestimmte Stellung, die einer ersten Blickrichtung zugeordnet ist, und mindestens eine andere vorbestimmte Stellung enthält, die mindestens einer anderen Blickrichtung zugeordnet ist.

12. Vorrichtung zur Bestimmung mindestens eines Sichtparameters nach Anspruch 11, wobei die erste Blickrichtung einer geraden waagrechten Richtung vor der Person entspricht, und wobei die mindestens eine andere Blickrichtung einer Nahsicht-Blickrichtung entspricht, die bezüglich der Waagrechten geneigt ist.

13. Vorrichtung zur Bestimmung mindestens eines Sichtparameters nach einem der Ansprüche 1 bis 12, wobei die ophthalmologischen Messeinrichtungen in der Lage sind, mindestens einen Sichtparameter der Art Sphäre, Zylinder, Achse, Aberration höherer Ordnung, Keratometrie und/oder korneale Topographie, und/oder Parameter des Pupillendurchmessers in einer ersten Blickrichtung und in mindestens einer anderen Blickrichtung, und/oder eine Differenz zwischen einem in der ersten Blickrichtung gemessenen und einem in der mindestens einen anderen Blickrichtung gemessenen Sichtparameter zu messen und aufzuzeichnen.

14. Vorrichtung zur Bestimmung mindestens eines Sichtparameters nach einem der Ansprüche 1 bis 13, wobei das ophthalmologische Messgerät ein binokulares Gerät ist, das eine erste Messachse, die dem rechten Auge der Person zugeordnet ist, und eine zweite Messachse hat, die dem linken Auge der Person zugeordnet ist, und wobei das optische Ausrichtsystem aus einem ersten optischen Ausrichtsystem und einem zweiten optischen Ausrichtsystem besteht, wobei das erste optische Ausrichtsystem dazu bestimmt ist, zwischen dem binokularen ophthalmologischen Messgerät und dem rechten Auge der Person angeordnet zu werden, und das zweite optische Ausrichtsystem dazu bestimmt ist, zwischen dem binokularen ophthalmologischen Messgerät und dem linken Auge der Person angeordnet zu werden.

## Claims

1. A device for determining at least one vision parameter of a subject along a plurality of monocular or binocular sight directions of the subject, said device comprising:
- a binocular or monocular ophthalmological measuring apparatus comprising ophthalmological means for measuring the vision parameter to be determined, which means are able to emit an illuminating optical beam, and to receive a measuring optical beam along at least one measuring optical axis (2) aligned with a preset sight direction of the eye in question;
- visual stimulating means able to generate a stimulating optical beam along a stimulating optical axis aligned with said preset sight direction of the eye in question; and
- head-supporting means able to receive the head of a subject and to keep it in a set posture,
**characterized in that**:
there being defined an ellipse (3) having a first focal point (E) on said measuring optical axis (2) and a second focal point (Y), said device comprises:
- at least one optical aligning system intended to be placed between the ophthalmological measuring means (1) and the eye in question (10, 20), said optical aligning system being able to reflect said illuminating and measuring optical beams between the focal points (E, Y) of the ellipse (3); and
- adjusting means able to modify the relative position of said optical aligning system relative to the head-supporting means so as to bring the second focal point (Y) into the vicinity of the center of rotation of the eye in question of the subject,
- said optical aligning system comprising first reflective optical means (15, 16, 16-A, 16-C, 31-A, 31-B, 41-A, 41-B) and second reflective optical means (17, 18, 32, 42), said first reflective optical means being tangent to said ellipse (3) at a first point (A) in a first sight direction (11, 21) and at at least one other point (B, C) in at least one other sight direction (12, 13, 22, 23) of the eye in question, and said second reflective optical means (17, 18, 32, 42) being mounted so as to be rotatably movable about the first focal point (E) between a first position (18-A) in which the optical aligning system aligns said first sight direction (11, 21) with the measuring optical axis (2) and at least one other position (18-B, 18-C) in which the optical aligning system aligns said at least one other sight direction (12, 13, 22, 23) of the eye in question with the measuring optical axis (2).

2. The device for determining at least one vision parameter as claimed in claim 1, in which the first reflective optical means comprise a spherical mirror, a planar mirror, a plurality of planar mirrors (16-A, 16-C), a dichroic plate (31-A, 31-B) or a plurality of dichroic plates (31-A, 31-B, 41-A, 41-B).

3. The device for determining at least one vision parameter as claimed in claim 1 or claim 2, in which said first reflective optical means comprise a first mirror (15, 16), and in which said optical aligning system comprises means for moving the first mirror (15, 16), which means are able to move the first mirror (15, 16) along a preset trajectory as a function of the binocular or monocular sight direction of the subject.

4. The device for determining at least one vision parameter as claimed in claim 3, in which said means for moving the first mirror comprise a hinged system of link connecting rods, a cam and/or a mechanical guiding system.

5. The device for determining at least one vision parameter as claimed in either of claims 3 and 4, in which said preset trajectory is an elliptical trajectory, and in which said first mirror (15, 16) is oriented so as to be tangent to said elliptical trajectory.

6. The device for determining at least one vision parameter as claimed in one of claims 1 to 5, in which said first reflective optical means comprise a first mirror (16-A) having a first preset position tangent to said ellipse (3) at a first point (A), and a second mirror (16-C) having a second preset position tangent to said ellipse (3) at another point (C).

7. The device for determining at least one vision parameter as claimed in claim 1, in which said first reflective optical means comprise an ellipsoidal mirror, and said second reflective optical means comprise a second mirror having a non-zero optical power, so that the optical aligning system formed by the first and second mirrors is afocal to the first order.

8. The device for determining at least one vision parameter as claimed in one of claims 1 to 7, in which said second reflective optical means (17, 18, 32, 42) comprise a planar mirror.

9. The device for determining at least one vision parameter as claimed in one of claims 1 to 8, in which said aligning system comprises a means for orienting the second reflective optical means (17, 18, 32, 42), able to make the second reflective optical means (17, 18, 32, 42) pivot about the first focal point (E) of the ellipse (3) as a function of the binocular or monocular sight direction (11, 21, 12, 22, 13, 23).

10. The device for determining at least one vision parameter as claimed in one of claims 1 to 9, comprising means for inclining said optical aligning system, said inclining means being able to orient the plane of the ellipse (3) about an axis passing through its focal points.

11. The device for determining at least one vision parameter as claimed in one of claims 1 to 10, in which said optical aligning system comprises at least one first preset position associated with a first sight direction, and at least one other preset position associated with at least one other sight direction.

12. The device for determining at least one vision parameter as claimed in claim 11, in which said first sight direction corresponds to a horizontal direction straight in front of the subject, and in which said at least one other sight direction corresponds to a near-vision sight direction that is inclined relative to the horizontal.

13. The device for determining at least one vision parameter as claimed in one of claims 1 to 12, in which the ophthalmological measuring means are able to measure and record at least one vision parameter such as sphere, cylinder, axis, higher-order aberration, a keratometry and/or corneal topography type parameter, and/or pupil diameter in a first sight direction and in at least one other sight direction, and/or a difference between a vision parameter measured in said first sight direction and measured in said at least one other sight direction.

14. The device for determining at least one vision parameter as claimed in one of claims 1 to 13, in which said ophthalmological measuring apparatus is a binocular apparatus having a first measuring axis associated with the right eye of the subject and a second measuring axis associated with the left eye of the subject, and wherein said at least one optical aligning system consists in a first optical aligning system and a second optical aligning system, the first optical aligning system being intended to be placed between said binocular ophthalmological measuring apparatus and the right eye of the subject, and
- the second optical aligning system being intended to be placed between said binocular ophthalmological measuring apparatus and the left eye of said subject.
